# EUROPEAN PATENT SPECIFICATION

(11) **EP 1 294 369 B1**
(45) Date of publication and mention of the grant of the patent: **07.09.2005**
(21) Application number: 01934785.5
(22) Date of filing: 22.05.2001
(51) Int. Cl.: A61K 31/15, A61K 9/08

(54) **NOVEL FORMULATIONS OF $g(a)-2,4-DISULFOPHENYL-N-TERT-BUTYLNITRONE**
NEUE FORMULIERUNGEN VON -G(A)-2,4-DISULFOPHENYL-N-TERT-BUTYLNITRON
NOUVELLES FORMULATIONS DE $g(a)-2,4-DISULFOPHENYLE-N-TERT-BUTYLNITRONE

(30) Priority: 23.05.2000 SE 0001916
(43) Date of publication of application: 26.03.2003
(73) Proprietor: AstraZeneca AB, 151 85 Södertälje (SE)
(72) Inventor: Andersson, Mats J., S-168 65 Bromma (SE); Andersson, Mattias, S-151 85 Södertälje (SE); Eriksson, Patrik, S-151 85 Södertälje (SE)
(86) International application number: PCT/SE2001/001164
(87) International publication number: WO 2001/089507

(56) References cited:
- US-A- 5 780 510
- US-A- 6 107 315
- MICHAEL E. AULTON: 'Pharmaceutics: The science of dosage form design', 1988 XP002948165 * page 368 - page 379 *

## Description

### Field of the Invention

This invention relates to novel pharmaceutical formulations of α-(2,4-disulfophenyl)-*N-tert*-butylnitrone and pharmaceutically acceptable salts thereof, and the use of such formulations in the treatment of various diseases and conditions. Such compounds are alternatively named as 4-[(*tert*-butylimino)methyl]benzene-1,3-disulfonic acid N-oxide derivatives.

### Background of the Invention

U.S. patent 5,488,145 discloses α-(2,4-disulfophenyl)-*N*-*tert*-butylnitrone and pharmaceutically acceptable salts thereof. U.S. patent 5,475,032 discloses the use of such compounds in the treatment of stroke and of progressive central nervous system function loss conditions. U.S. patent 5,508,305 discloses the use of such compounds for ameliorating the side effects caused by oxidative damage resulting from antineoplastic disease treatment. Similar disclosures are also made in WO 95/17876. U.S. patent 5,780,510 discloses the use of these same compounds in the treatment of concussion.

For use in the treatment of conditions such as stroke, concussion, traumatic brain injury and CNS trauma, it is required that a pharmaceutically acceptable salt of α-(2,4-disulfophenyl)-*N*-*tert*-butylnitrone should be administered parenterally. It is particularly preferred that the compound should be administered by intravenous infusion. Standard aqueous formulations of α-(2,4-disulfophenyl)-*N*-*tert*-butylnitrone and pharmaceutically acceptable salts thereof suffer from the problem that they readily undergo decomposition. In particular, the shelf life of such formulations is unacceptably short. The present invention discloses certain pharmaceutical formulations based upon concentrated aqueous solutions of α-(2,4-disulfophenyl)-*N*-*tert*-butylnitrone disodium salt that solve the problems associated with decomposition and that are particularly suited for use in parenteral administrations.

### Disclosure of the Invention

In one aspect, the present invention provides a pharmaceutical formulation of a compound of general formula (I) 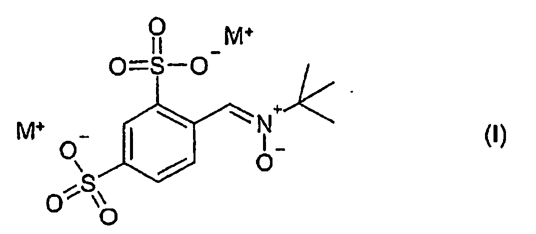 wherein
M⁺ represents Na⁺.

Aqueous solutions of α-(2,4-disulfophenyl)-*N*-*tert*-butylnitrone disodium salt undergo decomposition by at least two different pathways. 2,4-Disulphobenzaldehyde disodium salt (II) is a common product of these pathways.

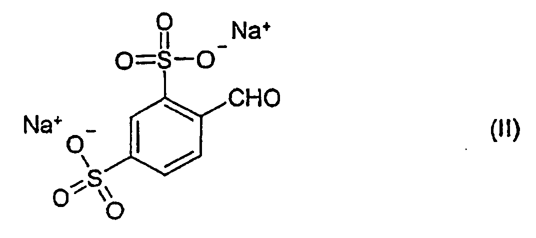

Without wishing to be bound by theory, it is apparent that one pathway for the decomposition involves hydrolysis of the nitrone functional group to yield the aldehyde (II) and N-*tert*-butylhydroxylamine as products. A second pathway involves an autoxidation process, possibly involving a free radical mediated degradation. In this pathway the same two products are formed initially but the N-*tert*-butylhydroxylamine subsequently undergoes further reactions to give other products. Autoxidation processes are known to be influenced by temperature, hydrogen ion concentration, trace metals, trace peroxides or light [K. Kasraian et al., Pharm. Dev. & Technol., 4(4), 475-480 (1999)]. For example, Fenton-type autoxidations are well known. Such autoxidations are typically initiated by the interaction of a metal, particularly iron, and molecular oxygen yielding a hydroxyl radical [B. Halliwell and J. Gutteridge, Biochem. J., 219, 1-14 (1984)].

Because of the complex nature of oxidative decompositions and because also in the present case there is a concurrent decomposition by hydrolytic cleavage, it is not obvious how the production of stable formulations of compounds of formula (I) could be achieved. It is recognised in the art that compounds that are susceptible to oxidative decompositions should be formulated at low (acidic) pH values so as to increase their resistance to oxidation. In particular, such decompositions are generally recognised to be minimised between pH 3 and 4 (Pharmaceutical Preformulation, ed. J.I. Wells, Ellis Horwood, 1988, page 166). However, in the present case use of a low pH results in an unacceptable acceleration of the rate of concomitant hydrolysis.

Studies were performed in order to ascertain which factors had a significant effect on the stability of aqueous formulations of α-(2,4-disulfophenyl)-*N*-*tert*-butylnitrone disodium salt. Factors investigated included pH, oxygen levels in and above the solution, the presence of trace metals and the addition of an antioxidant or of a chelating agent. In the first instance, decomposition was assessed by measuring the concentration of 2,4-disulphobenzaldehyde disodium salt (II) formed in the solution.

Trace metal analysis of various batches of α-(2,4-disulfophenyl)-*N*-*tert*-butylnitrone disodium salt indicated that the presence of even sub ppm levels of iron and also possibly of copper, chromium and aluminium might have an effect on the stability of subsequently prepared aqueous formulations. However, addition of disodium ethylenediamine tetraacetic acid (EDTA), a well known chelating agent, did not improve the stability of the aqueous formulation (Table 2). Use of the chelator resin Chelex-100® (Bio-Rad Laboratories) resulted in a small but significant reduction in the amount of the aldehyde (II) that was formed on storage (Example 3).

When sodium ascorbate, an antioxidant, was added to concentrated aqueous formulations of α-(2,4-disulfophenyl)-*N*-*tert*-butylnitrone disodium salt, the formation upon storage of the aldehyde (II) was reduced by almost half (Table 2). However, the solutions became discoloured and some precipitation occurred, thus ruling out a role for ascorbate as a means of reducing the level of decomposition. Surprisingly, similar levels of reduction of formation of the aldehyde (II) were achieved by the simple expedient of purging the concentrated aqueous solutions of α-(2,4-disulfophenyl)-*N*-*tert*-butylnitrone disodium salt with nitrogen gas (Tables 2 and 3).

In addition to purging the aqueous concentrate itself with an inert gas, it is also beneficial to reduce the volume of the headspace above the concentrate in the vial and to fill this space with an inert gas (Tables 4, 5 and 6). It is preferred that the headspace volume should be less than 30 % of the total maximum volume of the vial. It is more preferred that the headspace volume should be less than 20 % of the total maximum volume of the vial. For a standard 10 ml size pharmaceutical vial, the actual maximum total volume is 13 ml and it is convenient to use an actual fill volume of 10.7 ml. For a standard 20 ml size pharmaceutical vial, the actual maximum total volume is 25 ml and it is convenient to use an actual fill volume of 20.7 ml. The use of a standard 20 ml size pharmaceutical vial is preferred.

Most surprising was the fact that the stability of aqueous solutions of α-(2,4-disulfophenyl)-*N*-*tert*-butylnitrone disodium salt increased substantially as the concentration of α-(2,4-disulfophenyl)-*N*-*tert*-butylnitrone disodium salt in the solution increased. This stabilisation was apparent with respect to both a reduction in the amount of the aldehyde (II) that was formed and with respect to a reduction of further products resulting from an autoxidation pathway (Tables 8, 9 and 10).

A particular formulation according to the present invention therefore comprises a concentrated aqueous solution of α-(2,4-disulfophenyl)-*N*-*tert*-butylnitrone disodium salt wherein the concentration of the α-(2,4-disulfophenyl)-*N*-*tert*-butylnitrone disodium salt is in the range of 100 to 600 mg/ml. Preferred are formulations wherein the concentration is within the range of 200 to 400 mg/ml. Particularly preferred are formulations wherein the concentration is about 400 mg/ml. It is further preferred that such solutions are purged with and stored under an inert gas. Use of nitrogen as the inert gas is particularly preferred.

Such concentrated solutions do not require a buffer for further stabilisation. However, prior to administration to patients as intravenous infusions, such formulations are diluted with physiological saline. This process of dilution results in a drop in pH and the rate of decomposition of the resulting diluted solution thereby accelerates. In order to prevent this change in pH a buffer is needed. It is highly convenient that this buffer is included in the concentrated formulation rather than having to be added at the stage of dilution (Tables 11, 12 and 13).

Therefore, there is provided a concentrated aqueous formulation wherein the solution is buffered at pH 7 to 9.5. More preferably, the solution is buffered at about pH 8.5. Any physiologically acceptable buffer may be used. Preferably the buffer is a phosphate buffer. Thus, disodium hydrogen phosphate (5 to 50 mM) is added to the concentrate and the pH is adjusted to the required level by the addition of aqueous sodium hydroxide solution or of aqueous hydrochloric acid as appropriate.

In a further aspect, the present invention relates to a process for the preparation of novel formulations of pharmaceutically acceptable salts of α-(2,4-disulfophenyl)-*N*-*tert*-butylnitrone. In particular, a process for the preparation of novel formulations of α-(2,4-disulfophenyl)-*N*-*tert*-butylnitrone disodium salt.

In general terms, the process comprises dissolving a pharmaceutically acceptable salt of α-(2,4-disulfophenyl)-*N*-*tert*-butylnitrone in water or in a suitable aqueous buffer and thereafter, if necessary, adjusting the pH of the solution to within the range pH 7 to 9.5, and thereafter optionally degassing the solution using an inert gas such as nitrogen.

Preferably, the process comprises the steps of:
a) dissolving a suitable buffering agent such as disodium hydrogen phosphate in water for injection;
b) dissolving α-(2,4-disulfophenyl)-*N*-*tert*-butylnitrone disodium salt in said buffer solution;
c) checking the pH and then adjusting the pH to be within the range pH 7 to 9.5 by the addition of an appropriate amount of aqueous sodium hydroxide solution or of aqueous hydrochloric acid;
d) adding further water for injection to give the required final concentration of α-(2,4-disulfophenyl)-*N*-*tert*-butylnitrone disodium salt;
e) degassing the solution with nitrogen gas for a suitable period of time;
f) sterile filtering the solution through a 0.22 µm sterile filter into a pre-sterilised vessel; and
g) aseptically transferring the solution under nitrogen into individual vials that are subsequently sealed.

A particularly preferred process is the one specifically disclosed in Example 1.

In some circumstances it is particularly convenient to be able to present pharmaceutical formulations intended for parenteral administration in a multi-dose container. A multi-dose container is a container that permits the withdrawal of successive portions of the contents without changing the strength, quality or purity of the remaining portion. It is a regulatory requirement (European Pharmacopoeia 2001) that multi-dose aqueous injections contain a suitable antimicrobial preservative at an appropriate concentration except when the preparation itself has adequate antimicrobial properties. It is recognised in the art that pharmaceutical products that are aseptically filled (that is, ones that are terminally sterilised by filtration through a 0.22 µm filter) are extra sensitive to microbiological contamination during the manufacturing process. Both from a manufacturing point of view as well as for other safety reasons (for example, possible contamination due to damage caused during handling and storage of the product in the clinic), it is therefore considered to be a significant advantage if the drug formulation itself exhibits antimicrobial properties. Thus, if the pharmaceutical formulation itself fulfils the regulatory requirements relating to preservatives, the need for the addition of a separate preservative is abolished.

It is therefore a further advantage of the present invention that the concentrated aqueous formulations disclosed therein possess significant antimicrobial properties. Thus, the potential for formulations of α-(2,4-disulfophenyl)-*N*-*tert*-butylnitrone disodium salt to inhibit the growths of the following micro-organisms were assessed - *Ps. aeruginosa, S. aureus, Bur. cepacia, E. gergovia, E. coli, C. albicans* and *A. niger.*
As shown in Table 14, a concentrated aqueous formulation according to the present invention comprising α-(2,4-disulfophenyl)-*N*-*tert*-butylnitrone disodium salt (400 mg/ml) possesses considerable antimicrobial efficacy. Thus, for *Ps. aeruginosa, Bur. cepacia* and *E*. *gergovia*, very significant reductions (≥10³ fold) in colony forming units per ml (CFU/ml) are seen within 6 hours. And similar levels of effects are seen for *S. aureus* and *E. coli* within 24 hours, and for *C. albicans* within 48 hours. Detailed results are presented in Table 14, and comparative results for a formulation of α-(2,4-disulfophenyl)-*N*-*tert*-butylnitrone disodium salt (10 mg/ml) and for a buffer control are shown in Tables 15 and 16 respectively.

As shown in Table 7, dilute aqueous solutions of α-(2,4-disulfophenyl)-*N*-*tert*-butylnitrone disodium salt (0.9 mg/ml) undergo significant photodegradation when exposed to normal indoor lights at room temperature for 8 hours. The rate of photodegradation is reduced if the aqueous solution is buffered. More concentrated aqueous solutions (10 mg/ml) undergo photodegradation to a significantly reduced extent (Table 7). Under the same conditions an aqueous concentrate formulation according to one aspect of the present invention (400 mg/ml) underwent no photodegradation within the same time scale.

In a particularly preferred embodiment, the present invention provides a pharmaceutical formulation comprising a concentrated aqueous solution of α-(2,4-disulfophenyl)-*N*-*tert*-butylnitrone disodium salt (400 mg/ml) and disodium hydrogen phosphate (5 to 50 mM) at pH 8.5 purged with nitrogen and stored in sealed 20 ml glass vials with a small headspace volume and with the headspace filled with nitrogen. Even more preferably the disodium hydrogen phosphate is present at a concentration of about 10 mM. Such a formulation has an unexpectedly long shelf life of at least 24 months when stored refrigerated (temperature approximately 2 to 8 °C), and remains in useable condition for at least 6 months even when stored at room temperature.

The invention is illustrated but in no way limited by the following examples.

### Example 1

### Preparation of an aqueous concentrate formulation of α-(2,4-disulfophenyl)-N-tert-butylnitrone disodium salt

Disodium hydrogen phosphate dihydrate (186 g) was added to water for injection (60 kg). The mixture was stirred at a speed of 300 rpm until dissolution was complete (10 minutes). The pH of the solution was then 9.3. α-(2,4-Disulfophenyl)-*N*-*tert*-butylnitrone disodium salt (39.6 kg) was then added, and stirring was continued until this material was dissolved (20 minutes). The pH of the solution was then adjusted from 5.8 to 8.5 by the addition of 2M aqueous sodium hydroxide solution (604 ml). Further water for injection was added to give a final weight of 117.1 kg. Using these quantities a concentrate containing 400 mg/ml of α-(2,4-disulfophenyl)-*N*-*tert*-butylnitrone disodium salt is obtained. By varying the amount of the nitrone that is used, concentrates with concentrations in the range 50 to 600 mg/ml may be similarly prepared.

The solution was then degassed with nitrogen gas for 130 minutes (Table 1).

**Table 1**

| **Degassing Time (minutes)** | **Dissolved Oxygen (mg/L)** |
|---|---|
| 0 | 7.8 |
| 15 | 8.2 |
| 30 | 0.6 |
| 130 | 1.3 |

The solution was then sterile filtered using a 0.22 µm sterile filter into a pre-sterilised 400 L stainless steel vessel. The vessel was put under 10 to 15 kPa pressure using nitrogen gas.

The solution was filled aseptically into dry heat sterilised 10 ml or 20 ml glass vials using sterile filtered nitrogen gas that was purged into the vials both before and after filling. The fill volume was 10.5 ml or 20.7 ml respectively.

In-process control of residual oxygen in the vials was performed using a Toray Oxygen Analyser. Residual oxygen content in the headspace was 0.9 ± 0.1 % (n = 29).

### Example 2

### Relative influences of a chelating agent, an antioxidant, oxygen removal and pH on the stability of a concentrated aqueous solution of α-(2,4-disulfophenyl)-N-tert-butylnitrone disodium salt

The effects of several added factors were investigated in experiments designed using a multivariate technique. An aqueous solution of α-(2,4-disulfophenyl)-*N*-*tert*-butylnitrone disodium salt (100 mg/ml) containing less than 0.3% 2,4-disulphobenzaldehyde disodium salt (II) was placed in sealed 10 ml glass vials with a fill volume of 10 ml. The concentrations of the degradation products and particularly of 2,4-disulphobenzaldehyde disodium salt (II) were measured by a chromatographic method after accelerated storage conditions at + 40 °C and 75% relative humidity for two months.
The results are shown in Table 2.

**Table 2**

| **Added Factor** | **pH Range of Solution** | **Final Amount of Aldehyde (II) (area %)** |
|---|---|---|
| None (n = 5) | 7.0 to 8.2 | 2.20 ± 0.11 |
| Ascorbate (n = 3) | 7.0 to 7.3 | 1.32 ± 0.11 (p < 0.001) |
| EDTA (n = 5) | 7.0 to 8.8 | 2.27 ± 0.12 |
| Nitrogen purge (n = 3) | 7.0 to 8.6 | 1.34 ± 0.18 (p < 0.001) |

Values are mean ± standard deviation. n indicates the number of independent experiments. A t-test was performed to evaluate the significance of the different factors.

The pH-range studied in this experiment, pH 7 to 9, had no significant effect on the degree of decomposition.

### Example 3

### Effect of a chelator resin on the stability of a concentrated aqueous solution of α-(2,4-disulfophenyl)-N-tert-butylnitrone disodium salt

A concentrated aqueous solution of α-(2,4-disulfophenyl)-*N*-*tert*-butylnitrone disodium salt (100 mg/ml) and disodium hydrogen phosphate (5.3 mM) at pH 8.0 was passed overnight through a column of the chelator resin, Chelex-100®. The resulting solution was placed in portions (8 ml) into 10 ml glass vials which were then sealed. The starting level of the aldehyde (II) was 0.20%. After two months at + 40 °C and 75% relative humidity the concentration of the aldehyde (II) had increased to 2.3%. In a control experiment where the treatment with the resin was omitted, the level of the aldehyde (II) increased to 3.0%.

### Example 4

### Effects of purging with different air/nitrogen gas mixtures on the stability of a concentrated aqueous solution of α-(2,4-disulfophenyl)-N-tert-butylnitrone disodium salt

A concentrated aqueous solution of α-(2,4-disulfophenyl)-*N*-*tert*-butylnitrone disodium salt (400 mg/ml) and disodium hydrogen phosphate (5.3 mM) at pH 8.5 was purged with different levels of air/nitrogen gas mixtures. The solutions were stored in sealed 10 ml glass vials with a fill volume of 7 ml. The samples were stored for two months at + 40 °C and 75% relative humidity. The initial concentrated aqueous solution contained aldehyde (II) (0.25 area %) and related substances (0.56 area %). The results are shown in Table 3.

**Table 3**

| **% Air in Purge Gas Mixture** | **Final Amount of Aldehyde (II) (area %)** | **Final Amount of Related Substances (area %)** |
|---|---|---|
| 0 | 0.57 | 1.3 |
| 6.25 | 0.68 ± 0.08 (n = 2) | 1.5 ± 0.1 (n = 2) |
| 12.5 | 0.63 ± 0.01 (n = 2) | 1.4 |
| 25 | 0.77 | 1.6 |
| 50 | 0.93 | 1.9 |
| 100 | 1.27 | 2.4 |

### Example 5

### Evaluation of the importance of vial headspace volume

Another aspect of avoiding exposure to oxygen is to lower the volume of the headspace in the vials by increasing the fill volume.
A concentrated aqueous solution of α-(2,4-disulfophenyl)-*N*-*tert*-butyluitrone disodium salt (400 mg/ml) and disodium hydrogen phosphate (10.5 mM) at pH 8.5 was purged with nitrogen for 30 minutes. Either 8 ml or 13 ml portions of this solution were then placed in standard 10 ml glass vials (the maximum possible fill volume of a standard 10 ml glass vial is 13 ml). The headspace was not purged with nitrogen. The vials were sealed and stored at + 40 °C and 75% relative humidity for two months. The initial aldehyde level was 0.1%.
The results are shown in Table 4.

**Table 4**

| **Fill Volume (ml)** | **Final Amount of Aldehyde (II) (area %)** |
|---|---|
| 13 | 0.6 |
| 8 | 1.1 |

### Example 6

### Comparison of air or nitrogen filled headspaces

A 400 mg/ml concentrate of α-(2,4-disulfophenyl)-*N*-*tert*-butylnitrone disodium salt was prepared by adding α-(2,4-disulfophenyl)-*N*-*tert*-butylnitrone disodium salt (1500 g) to water for injection (2200 ml) containing dissolved disodium hydrogen phosphate dihydrate (3.51 g). The solution was then adjusted to pH 8.5 by the addition of 2M sodium hydroxide solution and then water for injection was added to give a final volume of 3750 ml. After preparation, the solution was purged with nitrogen for 90 minutes and then placed in 10 ml glass vials with a fill volume of 7.7 ml. Before stoppering the vials, the headspace was purged with nitrogen. Ten vials were sampled for oxygen content of the headspace and it was found to be less than 0.05%. The vials were stored either at + 5 °C at ambient humidity or at + 25 °C and 60% relative humidity.
A second batch of 400 mg/ml concentrate of α-(2,4-disulfophenyl)-*N*-*tert*-butylnitrone disodium salt buffered with 50 mM phosphate buffer was treated identically except that the headspace was filled with air not nitrogen and the fill volume was 10.5 ml.
The results are summarised in Table 5.

**Table 5**

| **Storage Time (months)** | **Amount of Aldehyde (II) (area %)** | | | |
|---|---|---|---|---|
| | **Nitrogen-filled Headspace** | | **Air-filled Headspace** | |
| | **+ 5 °C** | **+ 25 °C** | **+ 5 °C** | **+ 25 °C** |
| 0 | 0.2 | 0.2 | 0.3 | 0.3 |
| 3 | 0.2 | 0.4 | 0.3 | 0.7 |
| 6 | 0.2 | 0.3 | 0.5 | 1.0 |
| 12 | 0.3 | 0.5 | 0.4 | 0.7 |

Further data are shown in Table 6. For the 10 ml vial size, the fill volume was 10.7 ml; and for the 20 ml vial size, the fill volume was 20.7 ml.

**Table 6**

| | **Compositions** | | | | | | |
|---|---|---|---|---|---|---|---|
| **Concentration of α-(2,4-disulfophenyl)- N-tert-butylnitrone disodium salt (mg/ml)** | 50 | 50 | 100 | 400 | 400 | 400 | 400 |
| Vial size [ml] | 10 | 10 | 10 | 10 | 10 | 20 | 20 |
| Gas in headspace | air | N₂ | air | air | N₂ | air | N₂ |

| **Storage conditions** | **Amount of Aldehyde (II) (w/w %)** | | | | | | |
|---|---|---|---|---|---|---|---|
| Initial | 0.2 | 0.2 | 0.2 | 0.3 | 0.3 | 0.3 | 0.3 |
| 2 months at +40 °C | 2.8 | 1.4 | 1.8 | 0.8 | 0.5 | 0.7 | 0.5 |
| 3 months at +40 °C | 3.3 | 1.5 | N.A. | 0.9 | 0.6 | 0.8 | 0.5 |
| 6 months at +40 °C | 3.5 | 1.7 | N.A. | N.A. | N.A. | N.A. | N.A. |
| N.A. indicates not analysed. | | | | | | | |

### Example 7

### Evaluation of the photodegradation of aqueous solutions of α-(2,4-disulfophenyl)-N-tert-butylnitrone disodium salt

Diluted aqueous solutions of α-(2,4-disulfophenyl)-*N*-*tert*-butylnitrone disodium salt (0.9 mg/ml or 10 mg/ml) were tested for photostability under exposure to indoor light for 8 hours at room temperature. The lower concentration solutions were tested both with and without the addition of a carbonate buffer. A major photodegradation product was formed. The buffered formulation withstood photodegradation to a better extent than the unbuffered formulation. The formulation with α-(2,4-disulfophenyl)-*N*-*tert*-butylnitrone disodium salt (10 mg/ml) had the lowest rate of photodegradation.
Similar experiments using a 400 mg/ml aqueous concentrate of α-(2,4-disulfophenyl)-*N-tert*-butylnitrone disodium salt showed that in this case no degradation at all had occurred after 8 hours under the experimental conditions used.
The results are summarised in Table 7.

**Table 7**

| **Time (hours)** | **Amount of Photodegradation Product (area %)** | | |
|---|---|---|---|
| | **Concentration of α-(2,4- disulfophenyl)-*****N*****-*****tert*****- butylnitrone disodium salt 0.9 mg/ml Unbuffered** | **Concentration of α-(2,4- disulfophenyl)-*****N*****-*****tert*****- butylnitrone disodium salt 0.9 mg/ml Buffered** | **Concentration of α-(2,4- disulfophenyl)-*****N*****-*****tert*****- butylnitrone disodium salt 10 mg/ml Unbuffered** |
| 0 | 0.03 | 0 | 0 |
| 1 | 0.3 | 0.3 | 0.2 |
| 2 | 0. 8 | 0.6 | 0.2 |
| 3 | 1.2 | 0.9 | 0.4 |
| 4 | 1.8 | 1.3 | 0.5 |
| 5 | 2.5 | 1.6 | 0.6 |
| 6 | 2.9 | 2.0 | 0.8 |
| 7 | 3.5 | 2.3 | 0.8 |
| 8 | 4.0 | 2.7 | 0.9 |

### Example 8

### Effect of concentration on the stability of aqueous solutions of α-(2,4-disulfophenyl)-N-tert-butylnitrone disodium salt

In an initial experiment, aqueous solutions of three different concentrations of α-(2,4-disulfophenyl)-*N*-*tert*-butylnitrone disodium salt buffered with sodium hydrogen carbonate (50 mM) were dispensed into 20 ml glass vials, sealed, and then stored for 40 days at + 40 °C and 75% relative humidity. The particular batch of α-(2,4-disulfophenyl)-*N*-*tert*-butylnitrone disodium salt used had a high initial aldehyde content. The results are summarised in Table 8.

**Table 8**

| **Concentration of α-(2,4- disulfophenyl)-*****N*****-*****tert*****- butylnitrone disodium salt** **(mg/ml)** | **Initial Amount of Aldehyde (II)** **(Area %)** | **Final Amount of Aldehyde (II)** **(Area %)** |
|---|---|---|
| 200 | 1.7 | 3.5 |
| 300 | 1.7 | 3.2 |
| 400 | 1.8 | 2.9 |

In a second study unbuffered aqueous solutions of α-(2,4-disulfophenyl)-*N*-*tert*-butylnitrone disodium salt (concentration either 100 mg/ml or 200 mg/ml) were dispensed into 50 ml glass vials and stored at + 5 °C.
The results are summarised in Table 9.

**Table 9**

| **Storage Time** **(months)** | **Concentrate (100 mg/ml)** | | **Concentrate (200 mg/ml)** | |
|---|---|---|---|---|
| | **Amount of Aldehyde (II)** **(Area %)** | **pH** | **Amount of Aldehyde (II)** **(Area %)** | **pH** |
| 0 | 0.2 | 7.4 | 0.2 | 7.6 |
| 1 | 0.5 | 7.5 | 0.4 | 7.6 |
| 3 | 1.0 | 7.3 | 0.6 | 7.4 |
| 6 | 1.6 | 7.1 | 0.8 | 7.4 |
| 12 | 1.6 | 6.9 | 1.1 | 6.9 |

In a third study aqueous solutions of α-(2,4-disulfophenyl)-*N*-*tert*-butylnitrone disodium salt (concentration either 200 mg/ml or 400 mg/ml) buffered with phosphate buffer (50 mM) were dispensed into 10 ml glass vials and stored at + 5 °C.
The results are summarised in Table 10.

**Table 10**

| **Storage Time** **(months)** | **Concentrate (200 mg/ml)** | | **Concentrate (400 mg/ml)** | |
|---|---|---|---|---|
| | **Amount of Aldehyde (II)** **(Area %)** | **pH** | **Amount of Aldehyde (II)** **(Area %)** | **pH** |
| 0 | 0.1 | 8.0 | 0.1 | 8.0 |
| 6 | 0.4 | 7.9 | 0.3 | 7.8 |
| 12 | 0.6 | 7.9 | 0.4 | 7.8 |
| 18 | 0.7 | 8.0 | 0.4 | 7.9 |

### Example 9

### Effect of pH on the stability of aqueous solutions of α-(2,4-disulfophenyl)-N-tert-butylnitrone disodium salt

The pH dependent degradation of aqueous solutions of α-(2,4-disulfophenyl)-*N*-*tert*-butylnitrone disodium salt has been extensively studied. In Table 11 is shown a comparison of an unbuffered solution of α-(2,4-disulfophenyl)-*N*-*tert*-butylnitrone disodium salt (4 mg/ml) compared to a solution of α-(2,4-disulfophenyl)-*N-tert*-butylnitrone disodium salt (4 mg/ml) buffered with phosphate (0.53 mM). Both solutions, which were obtained by appropriate dilution of a corresponding concentrate, were stored at room temperature under conditions that would reasonably simulate a diluted concentrate prepared ready for administration to patients.

**Table 11**

| **Storage Time** **(days)** | **Unbuffered Solution** | | **Buffered Solution** | |
|---|---|---|---|---|
| | **Amount of Aldehyde (II)** **(Area %)** | **pH** | **Amount of Aldehyde (II)** **(Area %)** | **pH** |
| 0 | 0.9 | 6.8 | 0.8 | 8.0 |
| 1 | 1.1 | 6.8 | 0.9 | 7.9 |
| 2 | 1.2 | 6.8 | 0.9 | 7.7 |
| 5 | 1.6 | 6.8 | 1.0 | 7.5 |
| 7 | 2.0 | 6.7 | 1.1 | 7.5 |

### Example 10

### The effect on pH of the dilution of aqueous concentrates of α-(2,4-disulfophenyl)-N-tert-butylnitrone disodium salt

Three batches of an aqueous concentrate of α-(2,4-disulfophenyl)-*N*-*tert*-butylnitrone disodium salt (400 mg/ml) were prepared and adjusted to pH 8.5. One concentrate was unbuffered and the other two concentrates were buffered with respectively 2.6 or 26 mM disodium hydrogen phosphate. In each case a 7 ml portion of the concentrate was transferred into 0.9% sodium chloride solution (500 ml) contained in a PVC bag. The bags were then stored at room temperature, protected from light, for 48 hours. Samples were taken out at 0, 24 and 48 hours and analysed.
The results are summarised in Table 12.

**Table 12**

| **Time (hours)** | **No Buffer** | | **Buffer strength in concentrate (2.6 mM)** | | **Buffer strength in concentrate (26 mM)** | |
|---|---|---|---|---|---|---|
| | **pH** | **Amount of Aldehyde (II) (Area %)** | **pH** | **Amount of Aldehyde (II) (Area %)** | **pH** | **Amount of Aldehyde (II) (Area %)** |
| 0 | 6.01 | 0.25 | 6.50 | 0.30 | 7.43 | 0.17 |
| 24 | 6.32 | 0.68 | 6.69 | 0.44 | 7.39 | 0.20 |
| 48 | 6.48 | 0.89 | 6.67 | 0.57 | 7.38 | 0.25 |

### Example 11

### Effect of pH on the stability of aqueous solutions and concentrates of α-(2,4-disulfophenyl)-N-tert-butylnitrone disodium salt

In a further study the stability of both buffered (sodium hydrogen carbonate) and unbuffered aqueous solutions and concentrates of α-(2,4-disulfophenyl)-*N*-*tert*-butylnitrone disodium salt stored at room temperature were compared. The results (Table 13) demonstrate that decomposition is both concentration dependent and also is more pronounced at lower pH values. The buffered solutions show no apparent concentration dependent decomposition due to the short storage time and moderate storage temperature.

**Table 13**

| **Storage Time** **(hours)** | **Concentration of α-(2,4- disulfophenyl)-*****N*****-*****tert*****- butylnitrone disodium salt (mg/ml)** | **Unbuffered** | | **Buffered** | |
|---|---|---|---|---|---|
| | | **Amount of Aldehyde (II)** **(Area %)** | **pH** | **Amount of Aldehyde (II)** **(Area %)** | **pH** |
| 0 | 7.5 | 1.0 | 5.8 | 0.6 | 8.1 |
| 48 | 7.5 | 2.4 | 6.3 | 0.7 | 8.7 |
| 0 | 75 | 1.1 | 6.1 | 0.6 | 8.0 |
| 48 | 75 | 1.9 | 6.7 | 0.7 | 8.1 |
| 0 | 150 | 1.0 | 6.1 | 0.6 | 7.9 |
| 48 | 150 | 1.7 | 6.7 | 0.7 | 7.8 |

### Example 12

### Antimicrobial efficacy of α-(2,4-disulfophenyl)-N-tert-butylnitrone disodium salt (400 mg/ml) compared to α-(2,4-disulfophenyl)-N-tert-butylnitrone disodium salt (10 mg/ml) and to control.

The antimicrobial efficacy was tested for three different solutions:
i) α-(2,4-disulfophenyl)-*N*-*tert*-butylnitrone disodium salt, 10 mg/ml;
ii) α-(2,4-disulfophenyl)-*N*-*tert*-butylnitrone disodium salt, 400 mg/ml; and
iii) a carbonate buffer control.

The tests were performed according to European Pharmacopoeia 2000, Chapter 5.1.3, pages 259 to 260. Seven 10 ml vials were inoculated, one per test organism. During the tests, the vials were stored at controlled room temperature and protected from light. At different time intervals, samples were withdrawn and after appropriate dilutions the numbers of viable microorganisms (colony forming units per ml, CFU/ml) were determined using standard plate count procedures.

Results for the three solutions are shown in Tables 14, 15 and 16 respectively.

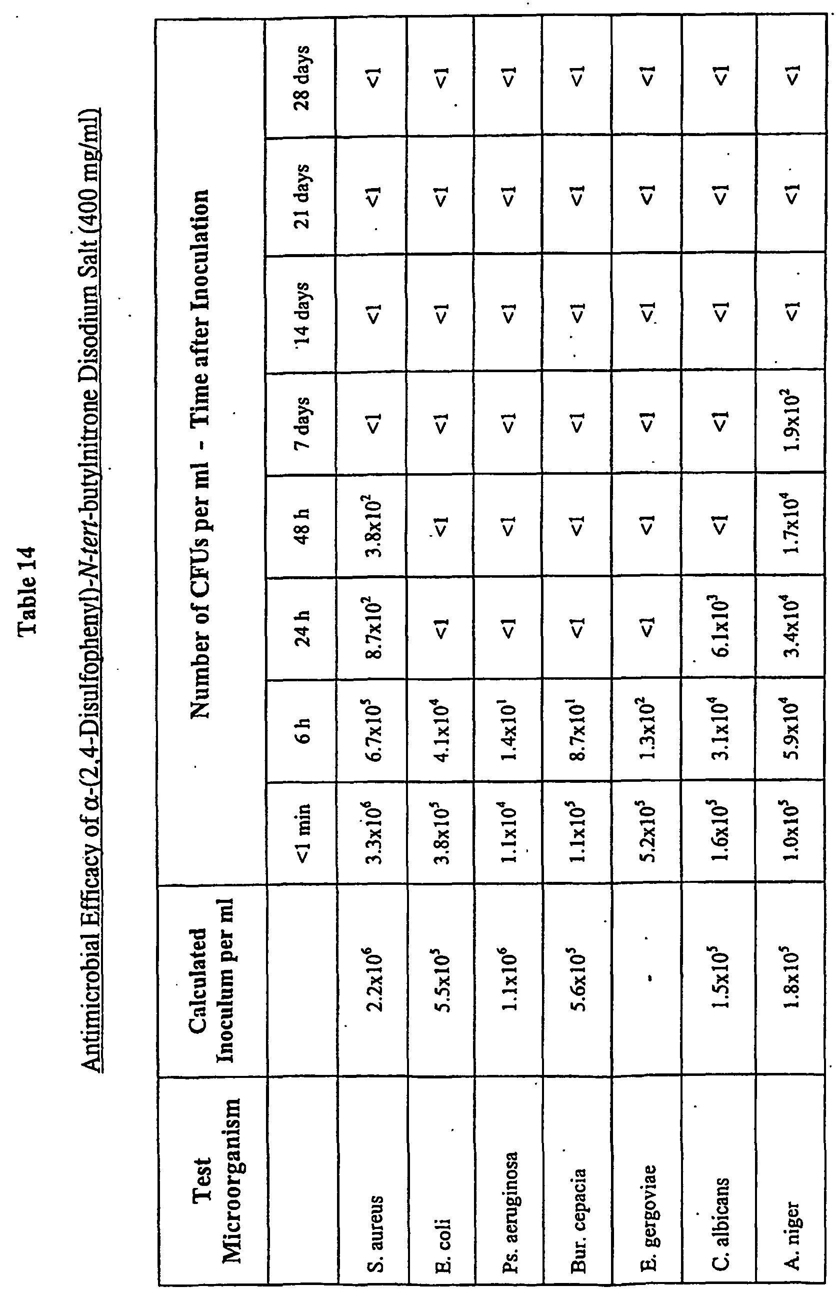

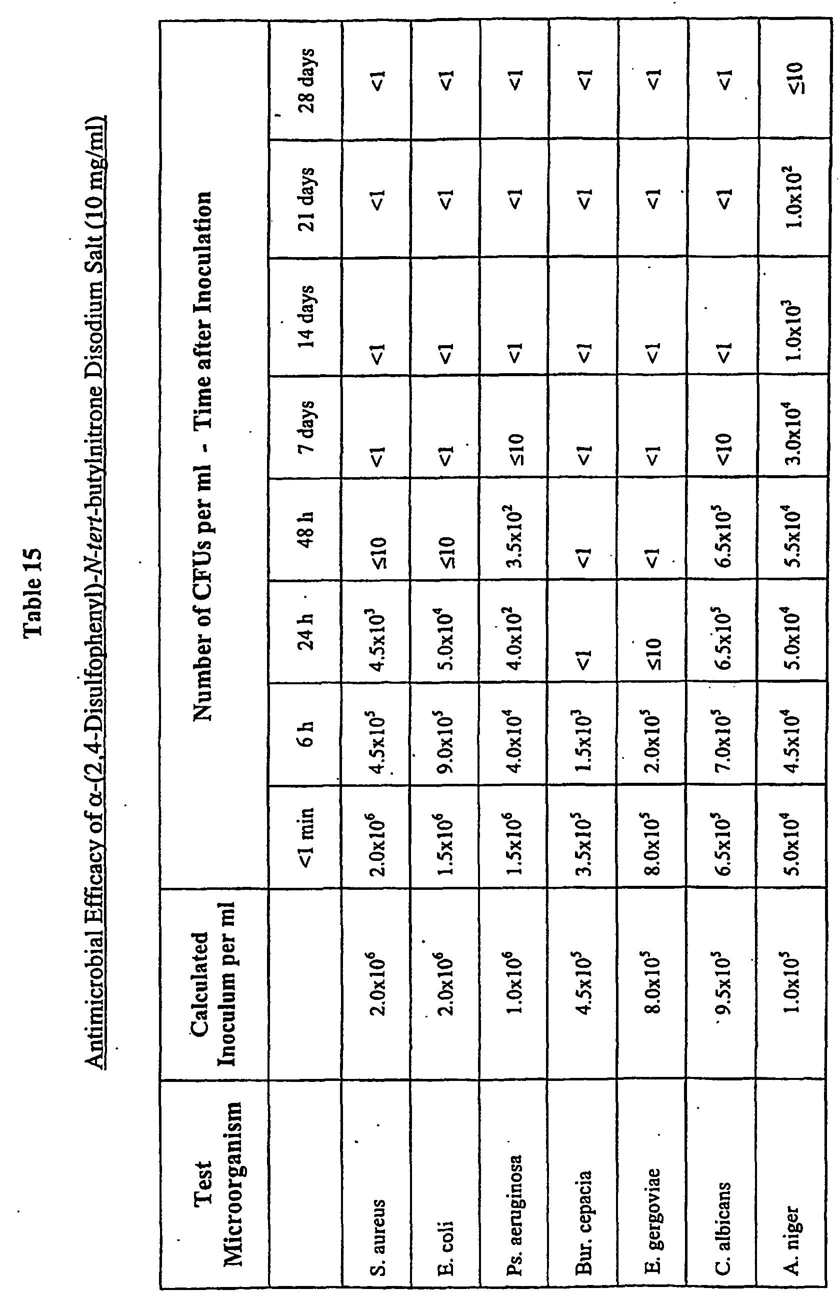

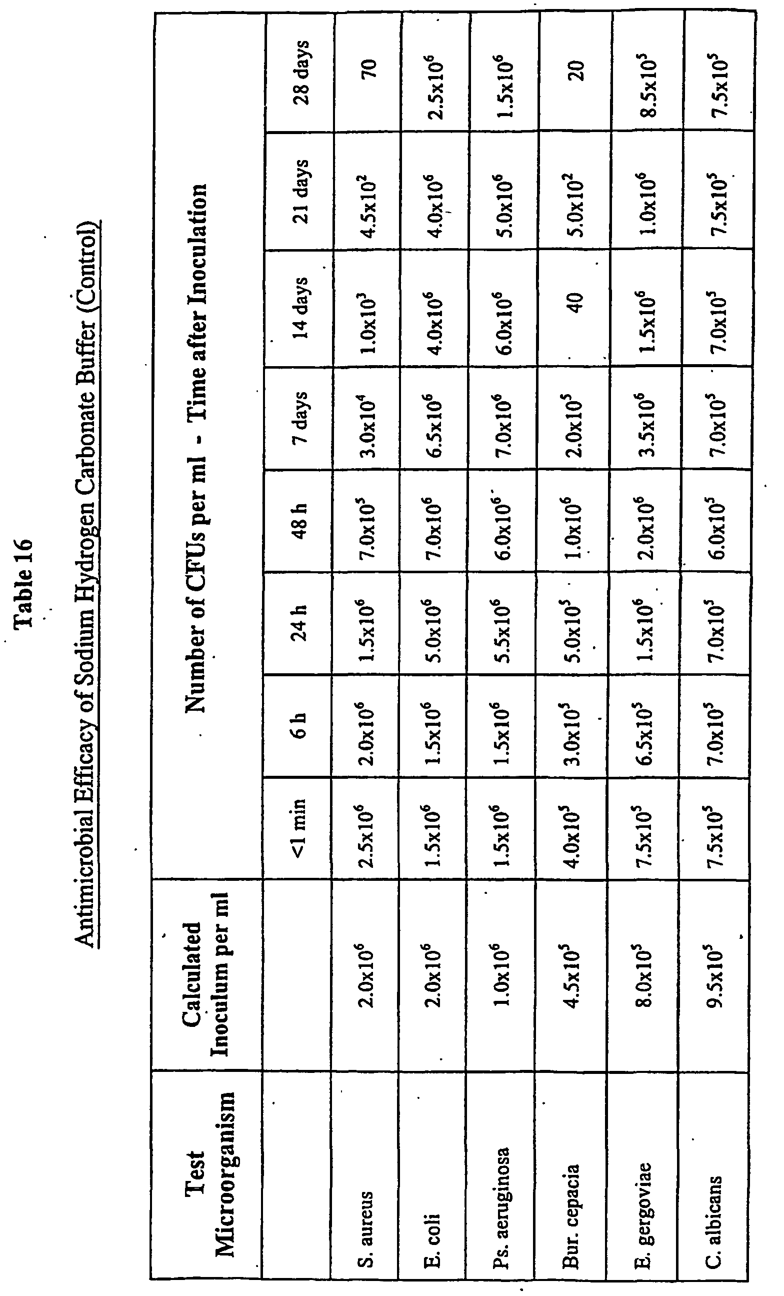

## Claims

1. A pharmaceutical formulation comprising a concentrated aqueous solution of α-(2,4-disulfophenyl)-*N*-*tert*-butylnitrone disodium salt and **characterised in that** the concentration is in the range 100 to 600 mg/ml and the pH is in the range 7 to 9.5.

2. A formulation according to Claim 1 wherein the concentration is 200 to 400 mg/ml.

3. A formulation according to Claim 1 wherein the concentration is about 400 mg/ml.

4. A formulation according to any one of Claims 1 to 3 wherein the solution is buffered using a physiologically acceptable buffer to within the pH range 7 to 9.5.

5. A formulation according to Claim 4 wherein the solution is buffered at about pH 8.5.

6. A formulation according to Claim 4 or Claim 5 wherein the buffer is a phosphate buffer.

7. A formulation according to any one of Claims 1 to 6 wherein the solution is purged with an inert gas.

8. A formulation according to any one of Claims 1 to 7 wherein the solution is purged with and stored under an inert gas.

9. A formulation according to any one of Claims 1 to 8 wherein the solution is stored in a sealed glass vial with a minimum headspace volume and the headspace is filled with an inert gas.

10. A formulation according to Claim 9 wherein the headspace volume within the sealed glass vial is less than 20% of the total maximum volume of the vial.

11. A formulation according to any one of claims 7 to 10 wherein the inert gas is nitrogen.

12. A process for the preparation of a formulation according to any one of Claims 1 to 7 which comprises dissolving α-(2,4-disulfophenyl)-*N*-*tert*-butylnitrone disodium salt in water for injection or in an appropriate aqueous buffer; and, if necessary, adjusting the pH of the solution to within the range pH 7 to 9.5; and thereafter optionally degassing the solution using an inert gas.

13. A process according to Claim 12 that comprises the steps of:
a) dissolving a suitable buffering agent such as disodium hydrogen phosphate in water for injection;
b) dissolving α-(2,4-disulfophenyl)-*N*-*tert*-butylnitrone disodium salt in said buffer solution;
c) checking the pH and then adjusting the pH to be within the range pH 7 to 9.5 by the addition of an appropriate amount of aqueous sodium hydroxide solution or of aqueous hydrochloric acid;
d) adding further water for injection to give the required final concentration of α-(2,4-disulfophenyl)-*N*-*tert*-butylaitrone disodium salt;
e) degassing the solution with nitrogen gas for a suitable period of time;
f) sterile filtering the solution through a 0.22 µm sterile filter into a pre-sterilised vessel; and
g) aseptically transferring the solution under nitrogen into individual vials that are subsequently sealed.

14. Use of a formulation according to any one of Claims 1 to 11 for the preparation of an intravenous infusion for the treatment of stroke.

15. Use of a formulation according to any one of Claims 1 to 11 for the preparation of an intravenous infusion for the treatment of concussion.

16. Use of a formulation according to any one of Claims 1 to 11 for the preparation of an intravenous infusion for the treatment of traumatic brain injury.

17. Use of a formulation according to any one of Claims 1 to 11 for the preparation of an intravenous infusion for the treatment of central nervous system trauma.

## Patentansprüche

1. Pharmazeutische Formulierung, bei der es sich um eine konzentrierte wäßrige Lösung von α-(2,4-Disulfophenyl)-N-tert.-butylnitron-dinatriumsalz handelt, **dadurch gekennzeichnet, daß** die Konzentration im Bereich von 100 bis 600 mg/ml liegt und der pH-Wert im Bereich von 7 bis 9,5 liegt.

2. Formulierung nach Anspruch 1, bei der die Konzentration 200 bis 400 mg/ml beträgt.

3. Formulierung nach Anspruch 1, bei der die Konzentration etwa 400 mg/ml beträgt.

4. Formulierung nach einem der Ansprüche 1 bis 3, bei der die Lösung mit einem physiologisch annehmbaren Puffer auf einen Wert im Bereich von pH 7 bis 9,5 gepuffert ist.

5. Formulierung nach Anspruch 4, bei der die Lösung auf etwa pH 8,5 gepuffert ist.

6. Formulierung nach Anspruch 4 oder 5, bei der es sich bei dem Puffer um einen Phosphatpuffer handelt.

7. Formulierung nach einem der Ansprüche 1 bis 6, bei der die Lösung mit Inertgas gespült wird.

8. Formulierung nach einem der Ansprüche 1 bis 7, bei der die Lösung mit Inertgas gespült wird und unter Inertgas aufbewahrt wird.

9. Formulierung nach einem der Ansprüche 1 bis 8, bei der die Lösung in einer verschlossenen Glasphiole mit minimalem Kopfraumvolumen aufbewahrt wird und der Kopfraum mit Inertgas gefüllt ist.

10. Formulierung nach Anspruch 9, bei der das Kopfraumvolumen in der verschlossenen Glasphiole weniger als 20% des gesamten Maximalvolumens der Phiole beträgt.

11. Formulierung nach einem der Ansprüche 7 bis 10, bei der es sich bei dem Inertgas um Stickstoff handelt.

12. Verfahren zur Herstellung einer Formulierung nach einem der Ansprüche 1 bis 7, bei dem man α-(2,4-Disulfophenyl)-N-*tert*.-butylnitron-dinatriumsalz in Wasser für Injektionszwecke oder einem geeigneten wäßrigen Puffer löst, gegebenenfalls den pH-Wert der Lösung auf einen Wert im Bereich von pH 7 bis 9,5 einstellt und danach gegebenenfalls die Lösung mit einem Inertgas entgast.

13. Verfahren nach Anspruch 12, bei dem man:
a) eine geeignete Puffersubstanz, wie Dinatriumhydrogenphosphat, in Wasser für Injektionszwecke löst;
b) in der Pufferlösung α-(2,4-Disulfophenyl)-N-*tert*.-butylnitron-dinatriumsalz löst;
c) den pH-Wert überprüft und dann durch Zugabe einer geeigneten Menge von wäßriger Natriumhydroxidlösung bzw. wäßriger Salzsäure den pH-Wert auf einen Wert im Bereich von pH 7 bis 9,5 einstellt;
d) durch Zugabe von weiterem Wasser für Injektionszwecke die geforderte Endkonzentration von α-(2,4-Disulfophenyl)-N-*tert*.-butylnitron-dinatriumsalz einstellt;
e) die Lösung über einen geeigneten Zeitraum mit Stickstoffgas entgast;
f) die Lösung über ein 0,22-µm-Sterilfilter steril in einen vorsterilisierten Behälter filtriert und
g) die Lösung unter Stickstoff aseptisch in einzelne Phiolen überführt, die danach verschlossen werden.

14. Verwendung einer Formulierung nach einem der Ansprüche 1 bis 11 zur Herstellung einer intravenösen Infusion zur Behandlung von Schlaganfällen.

15. Verwendung einer Formulierung nach einem der Ansprüche 1 bis 11 zur Herstellung einer intravenösen Infusion zur Behandlung von Gehirnerschütterungen.

16. Verwendung einer Formulierung nach einem der Ansprüche 1 bis 11 zur Herstellung einer intravenösen Infusion zur Behandlung von traumatischen Hirnverletzungen.

17. Verwendung einer Formulierung nach einem der Ansprüche 1 bis 11 zur Herstellung einer intravenösen Infusion zur Behandlung von Zentralnervensystem-Traumata.

## Revendications

1. Formulation pharmaceutique, comprenant une solution aqueuse concentrée d'α-(2,4-disulfophényl)-*N-tertio*-butylnitrone, sel disodique, et **caractérisée en ce que** la concentration se trouve dans la plage de 100 à 600 mg/ml et le pH se trouve dans la plage de 7 à 9,5.

2. Formulation selon la revendication 1, dans laquelle la concentration va de 200 à 400 mg/ml.

3. Formulation selon la revendication 1, dans laquelle la concentration est d'environ 400 mg/ml.

4. Formulation selon l'une quelconque des revendications 1 à 3, dans laquelle la solution est tamponnée à l'aide d'un tampon physiologiquement acceptable jusqu'au sein de la plage de pH allant de 7 à 9,5.

5. Formulation selon la revendication 4, dans laquelle la solution est tamponnée à environ pH 8,5.

6. Formulation selon la revendication 4 ou la revendication 5, dans laquelle le tampon est un tampon phosphate.

7. Formulation selon l'une quelconque des revendications 1 à 6, dans laquelle la solution est purgée par un gaz inerte.

8. Formulation selon l'une quelconque des revendications 1 à 7, dans laquelle la solution est purgée par, et stockée sous, un gaz inerte.

9. Formulation selon l'une quelconque des revendications 1 à 8, dans laquelle la solution est stockée dans une ampoule en verre fermée hermétiquement, ayant un volume d'espace de tête minimum, et l'espace de tête est rempli d'un gaz inerte.

10. Formulation selon la revendication 9, dans laquelle le volume d'espace de tête au sein de l'ampoule en verre fermée hermétiquement est inférieur à 20% du volume maximum total de l'ampoule.

11. Formulation selon l'une quelconque des revendications 7 à 10, dans laquelle le gaz inerte est l'azote.

12. Procédé de préparation d'une formulation selon l'une quelconque des revendications 1 à 7, comprenant la solubilisation d'α-(2,4-disulfophényl)-*N*-*tertio*-butylnitrone, sel disodique, dans de l'eau pour injection ou dans un tampon aqueux approprié ; et, le cas échéant, l'ajustement du pH de la solution jusqu'au sein de la plage de pH allant de 7 à 9,5 ; et ensuite le dégazage éventuel de la solution à l'aide d'un gaz inerte.

13. Procédé selon la revendication 12, comprenant les étapes :
a) de solubilisation d'un agent tampon convenable, tel que l'hydrogénophosphate disodique, dans de l'eau pour injection ;
b) de solubilisation d'α-(2,4-disulfophényl)-*N-tertio*-butylnitrone, sel disodique, dans ladite solution tampon ;
c) de vérification du pH, puis d'ajustement du pH pour être au sein de la plage de pH allant de 7 à 9,5, par l'addition d'une quantité appropriée d'une solution aqueuse d'hydroxyde de sodium ou d'acide chlorhydrique aqueux ;
d) d'addition de davantage d'eau pour injection pour donner la concentration finale requise en α-(2,4-disulfophényl)*-N-tertio*-butylnitrone, sel disodique ;
e) de dégazage de la solution par de l'azote gazeux pendant une période convenable ;
f) de filtration stérile de la solution à travers un filtre stérile de 0,22 µm dans un récipient préalablement stérilisé ; et
g) de transfert de manière aseptique de la solution sous azote dans des ampoules individuelles qui sont ensuite fermées hermétiquement.

14. Utilisation d'une formulation selon l'une quelconque des revendications 1 à 11, pour la préparation d'une perfusion intraveineuse destinée au traitement des accidents vasculaires cérébraux.

15. Utilisation d'une formulation selon l'une quelconque des revendications 1 à 11, pour la préparation d'une perfusion intraveineuse destinée au traitement de la commotion.

16. Utilisation d'une formulation selon l'une quelconque des revendications 1 à 11, pour la préparation d'une perfusion intraveineuse destinée au traitement de lésions cérébrales traumatiques.

17. Utilisation d'une formulation selon l'une quelconque des revendications 1 à 11, pour la préparation d'une perfusion intraveineuse destinée au traitement des traumatismes du système nerveux central.
